# EUROPEAN PATENT APPLICATION

(11) **EP 3 011 958 A1**
(43) Date of publication of application: **27.04.2016**
(21) Application number: 15177158.1
(22) Date of filing: 19.06.2009
(51) Int. Cl.: A61K 31/137, A61P 37/00

(54) **PAEDIATRIC COMPOSITIONS FOR TREATING MULTIPLE SCLEROSIS**

(30) Priority: 20.06.2008 US 132621 P
(62) Divisional of application: 09767784.3
(71) Applicant: Novartis AG, 4056 Basel (CH)
(72) Inventor: Kovarik, John, 4056 Basel (CH); Schmouder, Robert, Berkeley Heights, NJ New Jersey 07922 (US); Bastien, Marie-Claude, West Orange, NJ New Jersey 07052 (US); David, Oliver, 4056 Basel (CH); Karlsson, Goeril, 4056 Basel (CH); Bouillon, Thomas, 4056 Basel (CH)
(74) Representative: Wiessner, Michael

(57) **Abstract**

The present invention relates to pharmaceutical compositions comprising a 2-amino-2-[2-(4-C₂₋₂₀-alkyl-phenyl)ethyl]propane-1,3-diol compound or a pharmaceutically acceptable salt thereof, and to the use thereof for treating, preventing or delaying the progression of multiple sclerosis in a paediatric patient or a patient suffering from a specific condition.

## Description

The present invention relates to pharmaceutical compositions comprising a 2-amino-2-[2-(4-C₂₋₂₀-alkyl-phenyl)ethyl]propane-1,3-diol compound or a pharmaceutically acceptable salt thereof, and to the use thereof. In particular the present invention relates to the use of 2-amino-2-[2-(4-C₂₋₂₀-alkyl-phenyl)ethyl]propane-1,3-diol compound for treating, preventing or delaying the progression of multiple sclerosis (MS) in a patient, e.g. a paediatric patient or a patient suffering from a specific condition.

2-Amino-2-[2-(4-C₂₋₂₀-alkyl-phenyl)ethyl]propane-1,3-diol compounds are disclosed in EP-A-0627406, the relevant disclosure of which is incorporated herein by reference. On the basis of observed activity, the compounds have been found to be useful as immunosuppressants. Accordingly, the compounds may be useful in the treatment or prevention of various autoimmune conditions, including multiple sclerosis. A particular compound in this class is FTY720 (2-amino-2-[2-(4-octylphenyl)ethyl] propane-1,3-diol; fingolimod) which has the following structure:

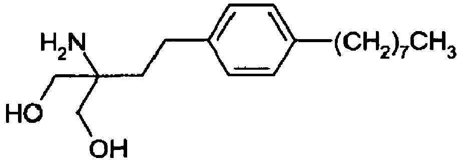

Also in this class of compounds is the compound FTY720 phosphate, which has the following structure:

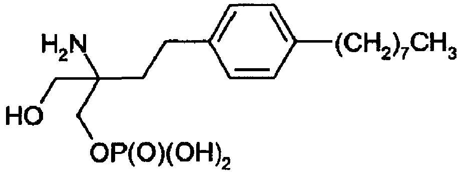

FTY720 acts as a modulator of sphingosine-1-phosphate (S1P) receptors, resulting in inhibition of the egress of lymphocytes from lymph nodes and Peyer's patches, and thereby reduces the recirculation of lymphocytes to blood and tissues including the Central Nervous System. FTY720 has demonstrated significant and consistent effects on Magnetic Resonance Imaging (MRI) measures of inflammation and relapses in study performed in adult patients with relapsing MS (Kappos, et al 2006], Oral fingolimod (FTY720)] for relapsing multiple sclerosis. N Engl J Med; 355(11):1124-1140).

As used herein, the term multiple sclerosis (MS) encompasses the different forms of the disease, including relapsing remitting, secondary progressive, primary progressive, and progressive relapsing multiple sclerosis.

Multiple sclerosis generally affects young adults, but it does also occur in adolescents and even children. Today, the therapies for the treatment of MS in paediatric patients which are approved are limited. They contain interferon beta and are administered by injection, e.g. intramuscularly or sub-cutaneously. Thus, there is a great medical need in multiple sclerosis to improve care for children with MS with new agents that are more effective than current first line therapies that are safe and offer better convenience than the currently available injectable therapies.

The present invention concerns the use of a pharmaceutical composition, e.g. pharmaceutical formulation, comprising about 1.25 mg or less as hereinbelow described, e.g. 0.5mg, of a 2-amino-2-[2-(4-C₂₋₂₀ alkylphenyl)ethyl]propane-1,3-diol compound (hereinafter referred to as "the compound"), e.g. FTY720 or FTY720 phosphate, or a pharmaceutically acceptable salt thereof. According to the invention, the composition may be administered orally to a patient in order to treat, prevent or delay of progression of multiple sclerosis in the patient, wherein the patient is e.g. a paediatric patient or is suffering from a chronic or recurrent condition selected from dyspnea, diarrhoea or nausea. In one embodiment of the invention, the composition may be administered once daily.

The compound may be, for example, a compound of the formula (I) as disclosed in EP-A-0627406 or a pharmaceutically acceptable salt thereof. The contents of this publication are incorporated herein by reference in their entirety.

The compound may be in the form of a phosphate, e.g. in which at least one of the hydroxy groups forming the diol portion of the molecule is phosphorylated.

The compound may be administered in free form or in pharmaceutically acceptable salt form. Such salts may be prepared in conventional manner and exhibit the same order of activity as the free compounds. Examples of pharmaceutically acceptable salts include salts with inorganic acids, such as hydrochloride, hydrobromide and sulfate, salts with organic acids, such as acetate, fumarate, maleate, benzoate, citrate, malate, methanesulfonate and benzenesulfonate salts, or, when appropriate, salts with metals, such as sodium, potassium, calcium and alumnium, salts with amines, such as triethylamine and salts with dibasic amino acids, such as lysine. Of particular mention are hydrochloride salts. The compounds and salts of the present invention encompass hydrate and solvate forms.

In a particular embodiment, the compound is FTY720, FTY720 phosphate or, in each case, a pharmaceutically acceptable salt, e.g. a hydrochloride salt, thereof. In another particular embodiment, the compound is the hydrochloride salt of FTY720. In another specific embodiment, the compound is FTY720 phosphate.

The pharmaceutical composition comprises about 1.25 mg, 0.5 mg or less of the compound or a pharmaceutically acceptable salt thereof. In an embodiment, the composition comprises about 1.25 mg of the compound or a pharmaceutically acceptable salt thereof. In another embodiment, the composition comprises about 1 mg or less of the compound or a pharmaceutically acceptable salt thereof. In another embodiment, the composition comprises about 0.5 mg or less of the compound or a pharmaceutically acceptable salt thereof. In yet another embodiment, the composition comprises about 0.5 mg of the compound or a pharmaceutically acceptable salt thereof. With regard to each of these embodiments, included are compositions in which the compound is FTY720, FTY720 phosphate or, in each case, a pharmaceutically acceptable salt, e.g. a hydrochloride salt, thereof.

The composition of the invention preferably contains 0.01 to 20% by weight of the compound, e.g. FTY720 or FTY720 phosphate, more preferably 0.1 to 10%, e.g. 0.5 to 5% by weight, based on the total weight of the composition.

The pharmaceutical composition may be a solid pharmaceutical composition in a form suitable for oral administration, e.g. a tablet or capsule. In another embodiment the composition may be liquid. The composition may be manufactured in a conventional manner, e.g. by mixing the compound, e.g. FTY720 or FTY720 phosphate, with a pharmaceutically acceptable carrier or diluent.

In a particular embodiment, the composition is a solid pharmaceutical composition comprising the compound, e.g. FTY720 or FTY720 phosphate, and a sugar alcohol. Compositions of this type are disclosed in WO 2004/089341, the contents of which are incorporated herein by reference. The solid compositions disclosed in this publication are particularly well suited to the oral administration of the compounds of the present invention, e.g. FTY720 or FTY720 phosphate. The compositions provide a convenient means of systemic administration of the compounds, do not suffer from the disadvantages of liquid compositions for injection or oral use, and have good physicochemical and storage properties. In particular, the compositions of the present invention may show a high level of uniformity in the distribution of the compound throughout the composition, as well as high stability. The compositions may therefore be manufactured on high speed automated equipment, and thus do not require hand encapsulation.

The sugar alcohol may act as a diluent, carrier, filler or bulking agent, and may suitably be mannitol, maltitol, inositol, xylitol or lactitol, preferably a substantially non-hygroscopic sugar alcohol, e.g. mannitol (D-mannitol). A single sugar alcohol may be used, or a mixture of two or more sugar alcohols, e.g a mixture of mannitol and xylitol, e.g. in a ratio of 1:1 to 4.1.

In a particularly preferred embodiment, the sugar alcohol is prepared from a spray-dried composition, e.g. mannitol composition, having a high specific surface area. The use of this type of mannitol composition may assist in promoting uniform distribution of the compound throughout the mannitol in the compositon. A higher surface area may be achieved by providing a sugar alcohol, e.g. mannitol, preparation consisting of particles having a smaller mean size and/or a rougher surface on each particle. The use of a spray- dried sugar alcohol, e.g. mannitol, e.g. with a mean particle size of 300 µm or less, has also been found to improve compressibility and hardness of tablets formed from the composition. Preferably the single point surface area of the sugar alcohol preparation, e.g. mannitol, is 1 to 7 m²/g, e g. 2 to 6 m²/g or 3 to 5 m²/g. The mannitol preparation may suitably have a mean particle size of 100 to 300 µm, e.g. 150 to 250 µm and a bulk density of 0.4 to 0.6 g/mL, e.g. 0.45 to 0.55 g/mL. A suitable high surface area mannitol is Parteck M200, available commercially from E. Merck.

The composition preferably contains 75 to 99.99% by weight of the sugar alcohol, more preferably 85 to 99.9%, e.g. 90 to 99.5% by weight, based on the total weight of the composition.

The composition preferably further comprises a lubricant. Suitable lubricants include stearic acid, magnesium stearate, calcium stearate, zinc stearate, glyceryl palmitostearate, sodium stearyl fumarate, canola oil, hydrogenated vegetable oil such as hydrogenated castor oil (e.g Cutina® or Lubriwax® 101), mineral oil, sodium lauryl sulfate, magnesium oxide, colloidal silicon dioxide, silicone fluid, polyethylene glycol, polyvinyl alcohol, sodium benzoate, talc, poloxamer, or a mixture of any of the above. Preferably the lubricant comprises magnesium stearate, hydrogenated castor oil or mineral oil. Colloidal silicon dioxide and polyethylene glycol are less preferred as the lubricant.

The composition preferably contains 0.01 to 5% by weight of the lubricant, more preferably 1 to 3% by weight, e.g. about 2% by weight, based on the total weight of the composition.

The composition may comprise one or more further excipients such as carriers, binders or diluents. In particular, the composition may comprise microcrystalline cellulose (e.g. Avicel®), methylcellulose, hydroxypropylcellulose, hydroxypropylmethylcellulose, starch (e.g corn starch) or dicalcium phosphate, preferably in an amount of from 0.1 to 90% by weight, e.g. 1 to 30% by weight, based on the total weight of the composition. Where a binder, e.g microcrystalline cellulose, methylcellulose, hydroxypropyl cellulose, hydroxypropylmethyl cellulose is used, it is preferably included in an amount of 1 to 8 %, e.g. 3 to 6% by weight, based on the total weight of the composition. The use of a binder increases the granule strength of the composition, which is particularly important for fine granulations. Microcrystalline cellulose and methylcellulose are particularly preferred where a high tablet hardness and/or longer disintegration time is required. Hydroxypropyl cellulose is preferred where faster distintegration is required. Where appropriate, xylitol may also be added as an additional binder, for example in addition to microcrystalline cellulose, e.g. in an amount up to 20% by weight of the sugar alcohol, e.g. xylitol.

In one embodiment, the composition further comprises a stabiliser, preferably glycine HCl or sodium bicarbonate. The stabiliser may be present in an amount of e.g. 0.1 to 30%, preferably 1 to 20% by weight.

The composition may be in the form of a powder, granule or pellets or a unit dosage form, for example as a tablet or capsule. The compositions of the present invention are well-adapted for encapsulation into an orally administrable capsule shell, particularly a hard gelatin shell.

Alternatively the compositions may be compacted into tablets. The tablets may optionally be coated, for instance with talc or a polysaccharide (e.g. cellulose) or hydroxypropylmethylcellulose coating.

Where a pharmaceutical capsule is in unit dosage form, each unit dosage may, for example, contain from about 0.5 to about 1.25 mg of the compound, e.g. FTY720 or FTY720 phosphate, or a pharmaceutically acceptable salt thereof.

The compositions of the invention may show good stability characteristics as indicated by standard stability trials, for example having a shelf life stability of up to one, two or three years, and even longer. Stability characteristics may be determined, e.g. by measuring decomposition products by HPLC analysis after storage for particular times, at particular temperatures, e.g. 20, 40 or 80 °C.

The pharmaceutical compositions of the present invention may be produced by standard processes, for instance by conventional mixing, granulating, sugar-coating, dissolving or lyophilizing processes. Procedures which may be used are known in the art, e.g. those described In L. Lachman et al. The Theory and Practice of Industrial Pharmacy, 3rd Ed, 1986, H Sucker et al, Pharmazeutische Technologie, Thieme, 1991, Hagers Handbuch der pharmazeutischen Praxis, 4th Ed. (Springer Veriag, 1971) and Remington's Pharmaceutical Sciences, 13th Ed., (Mack Publ., Co., 1970) or later editions.

In an embodiment, the pharmaceutical composition is produced by a process comprising:
(a) mixing the compound with a sugar alcohol;
(b) milling and/or granulating the mixture obtained in (a); and
(c) mixing the milled and/or granulated mixture obtained in (b) with a lubricant.

By using this process, a preparation having a good level of content and blend uniformity (i.e. a substantially uniform distribution of the compound throughout the composition), dissolution time and stability is obtained.

The compound, e.g. FTY720 or FTY720 phosphate, may optionally be micronized, and/or prescreened, e.g. with a 400 to 500 µm mesh screen, before step (a) in order to remove lumps. The mixing step (a) may suitably comprise blending the compound and the sugar alcohol, e.g. mannitol in any suitable blender or mixer for e.g. 100 to 400 revolutions.

The process may be carried out by dry mixing the components. In this embodiment the milling step (b) may suitably comprise passing the mixture obtained in (a) through a screen, which preferably has a mesh size of 400 to 500 µm. Process step (a) may comprise the step of mixing the total amount of the compound at first with a low amount of sugar alcohol, e.g. from 5 to 25% by weight of the total weight of sugar alcohol, in order to form a pre-mix. Subsequently the remaining amount of sugar alcohol is added to the pre-mix. Step (a) may also comprise the step of adding a binder solution, e.g. methylcellulose and/or xylitol, e.g. an aqueous solution, to the mixture. Alternatively the binder is added to the mix dry and water is added in the granulation step.

The milled mixture obtained in (b) may optionally be blended once more before mixing with the lubricant. The lubricant, e.g. magnesium stearate, is preferably pre-screened, e.g. with a 800 to 900 µm screen, before mixing.

Alternatively, a wet granulation process is employed. In this embodiment, the compound, e.g. FTY720 or FTY720 phosphate, is preferably first dry-mixed with the desired sugar alcohol, e.g. mannitol, and the obtained sugar alcohol/compound mixture is then dry-mixed with a binder such as hydroxypropyl cellulose or hydroxypropylmethyl cellulose. Water is then added and the mixture granulated, e.g. using an automated granulator. The granulation is then dried and milled.

If desirable, an additional amount of binder may be added in step (c) to the mixture obtained in (b).

The process may comprise a further step of tabletting or encapsulating the mixture obtained in (c), e.g. into a hard gelatin capsule using an automated encapsulation device. The capsules may be coloured or marked so as to impart an individual appearance and to make them instantly recognizable. The use of dyes can serve to enhance the appearance as well as to identify the capsules. Dyes suitable for use in pharmacy typically include carotinoids, iron oxides, and chlorophyll. Preferably, the capsules of the invention are marked using a code.

The pharmaceutical composition may comprise or be administered in conjunction with another active pharmaceutical ingredient, e.g. an immunomodulating or anti-inflammatory agent. For example, the compound may be used in combination with calcineurin inhibitors, e.g. cyclosporin A, cyclosporin G, FK-506, ABT-281, ASM 981; an mTOR inhibitor, e.g. rapamycin, 40-O-(2-hydroxy)ethyl-rapamycin, CCI779, ABT578 or AP23573 etc.; corticosteroids; cyclophosphamide; azathioprene; methotrexate; another S1P receptor agonist, e.g. FTY 720 or an analogue thereof; leflunomide or analogs thereof; mizoribine; mycophenolic acid; mycophenolate mofetil; 15-deoxyspergualine or analogs thereof; immunosuppressive monoclonal antibodies, e.g., monoclonal antibodies to leukocyte receptors, e.g., MHC, CD2, CD3, CD4, CD 11a/CD18, CD7, CD25, CD 27, B7, CD40, CD45, CD58, CD 137, ICOS, CD150 (SLAM), OX40, 4-1BB or their ligands, e.g. CD154; or other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a mutant thereof, e.g. LEA29Y, or other adhesion molecule inhibitors, e.g. mAbs or low molecular weight inhibitors including LFA-1 antagonists, Selectin antagonists and VLA-4 antagonists. Dosages of the co-administered immunomodulating or anti-inflammatory agent will of course vary depending on the type of co-drug employed, on the condition to be treated and so forth.

The pharmaceutical compositions of the present invention may be useful in the treatment and prevention of multiple sclerosis in a paediatric patient. The term "paediatric patient" as used herein refers to a patient under the age of 18 years, e.g. under the age of 16 years. Included are patients ranging from 0 to 17 years, e.g. from 0 to 15 years, from 11 to 16 years, in particular from 5 to 12 years, or from 10 to 12 years.

According to the invention, daily dosage of the compound, e.g. FTY720 or FTY720 phosphate, is about 1.25 mg or less, e.g. is about 1.25 mg to about 0.01 mg, e.g. is about 1.25 mg, e.g. 1.20 mg, e.g. 1.15 mg, e.g. 1.10 mg, e.g. 1.05mg, e.g. 1.00 mg, e.g. 0.95mg, e.g. 0.90 mg, e.g. 0.85mg, e.g. 0.80 mg, e.g. 0.75 mg, e.g. 0.70 mg, e.g. 0.65 mg, e.g. 0.60 mg, e.g. 0.55 mg, e.g. 0.50 mg, e.g. 0.45 mg, e.g. 0.40 mg, e.g. 0.35 mg, e.g. 0.30 mg, e.g. 0.25mg, e.g. 0.20 mg, e.g. 0.15 mg, e.g. 0.125 mg, e.g. 0.12 mg, e.g. 0.115 mg, e.g. 0.11mg, e.g. 105 mg, e.g. 0.1 mg, e.g. 0.055 mg, e.g. 0.05 mg, e.g. 0.045 mg, e.g. 0.04 mg, e.g. 0.035 mg, e.g. 0.03 mg, e.g. 0.025 mg, e.g. 0.02 mg, e.g. 0.01 mg. Preferably the daily dosage of the compound, e.g. FTY720 or FTY720 phosphate, is 0.5mg. With regard to each of these individual embodiments, included are administration of daily dosage of the compound which is FTY720, FTY720 phosphate or, in each case, a pharmaceutically acceptable salt, e.g. a hydrochloride salt, thereof. In particular are included the below mentioned daily dosages of FTY720 phosphate or the hydrochloride salt of FTY720.

In a specific embodiment the daily dosage of FTY720, FTY720 phosphate or, in each case, a pharmaceutically acceptable salt, e.g. a hydrochloride salt, is about 0.5 mg, or about 0.25mg, or about 0.125 mg. In another embodiment the daily dosage of FTY720 phosphate or the hydrochloride salt of FTY720 is about 0.5 mg, or about 0.25mg, or about 0.125 mg.

Accordingly, the present invention provides:
1. A pharmaceutical composition comprising about 1.25 mg or less as herein above described, e.g. about 0.5 mg or less, e.g. about 0.5 mg of the compound, e.g. FTY720 or FTY720 phosphate, or a pharmaceutically acceptable salt thereof, for use in the treatment, prevention or delay of progression of multiple sclerosis in a patient, wherein the composition is administered orally, e.g. once daily, and wherein the patient is a paediatric patient.
2. Use of a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, in the treatment, prevention or delay of progression of multiple sclerosis in a patient, wherein the composition is administered orally, e.g. once daily, and wherein the patient is a paediatric patient.
3. Use of a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, for the manufacture of a medicament for the treatment, prevention or delay of progression of multiple sclerosis in a patient, wherein the medicament is administered orally, e.g. once daily, and wherein the patient is a paediatric patient.
4. A package comprising a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof; and a label bearing instructions to use of the composition for the treatment, prevention or delay of progression of multiple sclerosis in a patient, wherein the composition is to be administered orally, e.g. once daily, and wherein the patient is a paediatric patient.
5. A method of treating, preventing or delaying the progression of multiple sclerosis in a patient, which comprises administering a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, wherein the composition is administered orally, e.g. once daily, and wherein the patient is a paediatric patient.

In an embodiment, the composition for administration to the paediatric patient comprises about 1.25 mg of the compound or a pharmaceutically acceptable salt thereof. In another embodiment, the composition comprises about 1 mg or less of the compound or a pharmaceutically acceptable salt thereof. In another embodiment, the composition comprises about 0.5 mg or less of the compound or a pharmaceutically acceptable salt thereof. In yet another embodiment, the composition comprises about 0.5 mg of the compound or a pharmaceutically acceptable salt thereof. With regard to each of these embodiments, included are compositions in which the compound is FTY720, FTY720 or, in each case, a pharmaceutically acceptable salt, e.g. a hydrochloride salt, thereof.

The invention includes the treatment, prevention of delay of progression of relapsing/remitting multiple sclerosis in a paediatric patient. The paediatric patient may be one suffering from a condition selected from dyspnea, diarrhoea and nausea. The condition may be chronic or recurrent.

The pharmaceutical compositions of the invention may also be useful in the treatment and prevention of multiple sclerosis in a patient suffering from a chronic or recurrent condition selected from dyspnea, diarrhoea and nausea. A chronic condition may, for example, last for about 1 week or more, e.g. 2 weeks or more, in particular about 4 weeks or more. A patient suffering from a recurrent condition may experience multiple episodes of the condition each year, e.g. at least biannually, in particular at least tri-annually, wherein episodes are separated by a period of intermission.

Accordingly, the present invention also provides:
1. A pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, for use in the treatment, prevention or delay of progression of multiple sclerosis in a patient, wherein the composition is to be administered orally, e.g. once daily, and wherein the patient is suffering from a chronic or recurrent condition selected from dyspnea, diarrhoea and nausea.
2. Use of a pharmaceutical composition comprising about 1.25 mg or less, e.g. about 0.5 mg or less, e.g. about 0.5 mg, of the compound, e.g. FTY720 or FTY720 phosphate, or a pharmaceutically acceptable salt thereof, in the treatment, prevention or delay of progression of multiple sclerosis in a patient, wherein the composition is to be administered orally, e.g. once daily, and wherein the patient is suffering from a chronic or recurrent condition selected from dyspnea, diarrhoea and nausea.
3. Use of a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, for the manufacture of a medicament for the treatment, prevention or delay of progression of multiple sclerosis in a patient, wherein the medicament is to be administered orally, e.g. once daily, and wherein the patient is suffering from a chronic or recurrent condition selected from dyspnea, diarrhoea and nausea.
4. A package comprising a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof; and a label bearing instructions to use of the composition for the treatment, prevention or delay of progression of multiple sclerosis in a patient, wherein the composition is to be administered orally, e.g. once daily, and wherein the patient is suffering from a chronic or recurrent condition selected from dyspnea, diarrhoea and nausea.
5. A method of treating, preventing or delaying the progression of multiple sclerosis in a patient, which comprises administering a pharmaceutical composition comprising about 1.25 mg or less, e.g. about 0.5 mg or less, e.g. about 0.5 mg of the compound, e.g. FTY720 or FTY720 phosphate, or a pharmaceutically acceptable salt thereof, wherein the composition is to be administered orally, e.g. once daily, and wherein the patient is suffering from a chronic or recurrent condition selected from dyspnea, diarrhoea and nausea.

In an embodiment, the pharmaceutical composition administered to the patient suffering from the chronic or recurrent condition comprises about 1.25 mg of the compound or a pharmaceutically acceptable salt thereof. In another embodiment, the composition comprises about 1 mg or less of the compound or a pharmaceutically acceptable salt thereof. In another embodiment, the composition comprises about 0.5 mg or less of the compound or a pharmaceutically acceptable salt thereof. In yet another embodiment, the composition comprises about 0.5 mg of the compound or a pharmaceutically acceptable salt thereof. With regard to each of these embodiments, included are compositions in which the compound is FTY720, FTY720phosphate or, in each case, a pharmaceutically acceptable salt, e.g. a hydrochloride salt, thereof.

The invention includes the treatment, prevention of delay of progression of relapsing/remitting multiple sclerosis in a patient suffering from one of the aforementioned chronic or recurrent conditions. The patient may be suffering from one or more of the aforementioned conditions and may be a paediatric patient.

According to the invention, 2-amino-2-[2-(4-C₂₋₂₀-alkyl-phenyl)ethyl]propane-1,3-diol compound, e.g. FTY720, FTY720 phosphate or, in each case, a pharmaceutically acceptable salt, e.g. a hydrochloride salt, thereof, may be co-administered, e.g. concomitantly or in sequence, with at least one co-agent shown to have clinical activity against at least one symptom of a demyelinating disease.

The co-agent b) may be selected from the following groups of compounds:
i) Interferons, e.g. pegylated or non-pegylated α-interferons, or β-interferons, e.g. interferon beta-1a or interferon beta-1 b, or τ-interferons, e.g. administered by subcutaneous, intramuscular or oral routes, preferably β-interferons;
ii) An altered peptide ligand such as Glatiramer, e.g. in the acetate form;
iii) Immunosuppressants with optionally antiproliferative/antineoplastic activity, e.g. mitoxantrone, methotrexate, azathioprine, cyclophosphamide, or steroids, e.g. methylprednisolone, prednisone or dexamethasone, or steroid-secreting agents, e.g. ACTH;
iv) Adenosine deaminase inhibitors, e.g. cladribine;
v) IV immunoglobulin G (e.g. as disclosed in Neurology, 1998, May 50(5):1273-81
vi) Monoclonal antibodies to various T-cell surface markers, e.g. natalizumab (ANTEGREN®) or alemtuzumab;
vii) TH2 promoting cytokines, e.g. IL-4, IL-10, or compounds which inhibit expression of TH1 promoting cytokines, e.g. phosphodiesterase inhibitors, e.g. pentoxifylline;
viii) Antispasticity agents including baclofen, diazepam, piracetam, dantrolene, lamotrigine, rifluzole, tizanidine, clonidine, beta blockers, cyproheptadine, orphenadrine or cannabinoids;
ix) AMPA glutamate receptor antagonists, e.g. 2,3-dihydroxy-6-nitro-7-sulfamoylbenzo(f)quinoxaline, [1,2,3,4,-tetrahydro-7-morpholin-yl-2,3-dioxo-6-(trifluoromethyl)quinoxalin-1-yl]methylphosphonate, 1-(4-aminophenyl)-4-methyl-7,8-methylene-dioxy-5H-2,3-benzodiazepine, or (-)1-(4-aminophenyl)-4-methyl-7,8-methylene-dioxy-4,5-dihydro-3-methylcarbamoyl-2,3-benzodiazepine;
x) Inhibitors of VCAM-1 expression or antagonists of its ligand, e.g. antagonists of the α4β1 integrin VLA-4 and/or alpha-4-beta-7 integrins, e.g. natalizumab (ANTEGREN®);
xi) Anti-Macrophage migration inhibitory factor (Anti-MIF);
xii) Cathepsin S inhibitors;
xiii) mTor inhibitors.

Cathepsin S inhibitors include e.g.:
a) a compound as disclosed in WO 03/20721, e.g. a compound of formula: 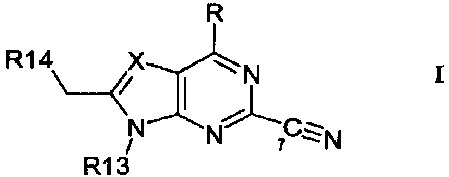 wherein
   R is H, -R2, -OR2 or NR1 R2,
   wherein R1 is H, lower alkyl or C₃ to C₁₀ cycloalkyl, and
   R2 is lower alkyl or C₃ to C₁₀ cycloalkyl, and
   wherein each of R1 and R2 independently, is optionally substituted by halo, hydroxy, lower alkoxy, CN, NO₂, or optionally mono- or di-lower alkyl substituted amino;
   X is =N- or =C(Z)-,
   wherein Z is H, -C(O)-NR3R4, -NH-C(O)-R3, -CH₂-NH-C(O)-R3, -C(O)-R3, -S(O)-R3,-S(O)₂-R3,-CH₂-C(O)-R3, -CH₂-NR3R4, -R4, -C≡C-CH₂-R5, N-heterocyclyl, N-heterocyclyl-carbonyl, or -C(P)=C(Q)-R4
   wherein
   each of P and Q, independently, is H, lower alkyl or aryl,
   R3 is aryl, aryl-lower alkyl, C₃-C₁₀cycloalkyl, C₃-C₁₀cycloalkyl-lower alkyl, heterocyclyl or heterocyclyl-lower alkyl,
   R4 is H, aryl, aryl-lower alkyl, aryl-lower-alkenyl, C₃-C₁₀cycloalkyl, C₃-C₁₀cycloalkyl-lower alkyl, heterocyclyl or heterocyclyl-lower alkyl, or
   wherein R3 and R4 together with the nitrogen atom to which they are joined to form an N-heterocyclyl group,
   wherein N-heterocyclyl denotes a saturated, partially unsaturated or aromatic nitrogen containing heterocyclic moiety attached via a nitrogen atom thereof having from 3 to 8 ring atoms optionally containing a further 1, 2 or 3 heteroatoms selected from N, NR6, O, S, S(O) or S(O)₂ wherein R6 is H or optionally substituted (lower alkyl, carboxy, acyl (including both lower alkyl acyl, e.g. formyl, acetyl or propionyl, or aryl acyl, e.g. benzoyl), amido, aryl, S(O) or S(O)₂), and wherein the N-heterocyclyl is optionally fused in a bicyclic structure, e.g. with a benzene or pyridine ring, and wherein the N-heterocyclyl is optionally linked in a spiro structure with a 3 to 8 membered cycloalkyl or heterocyclic ring wherein the heterocyclic ring has from 3 to 10 ring members and contains from 1 to 3 heteroatoms selected from N, NR6, O, S, S(O) or S(O)₂ wherein R6 is as defined above), and
   wherein heterocyclyl denotes a ring having from 3 to 10 ring members and containing from 1 to 3 heteroatoms selected from N, NR6, O, S, S(O) or S(O)₂ wherein R6 is as defined above), and
   wherein each of R3 and R4, independently, is optionally substituted by one or more groups, e.g. 1-3 groups, selected from halo, hydroxy, oxo, lower alkoxy, CN or NO₂, or optionally substituted (optionally mono- or di-lower alkyl substituted amino, aryl, aryl-lower alkyl, N-heterocyclyl or N-heterocyclyl-lower alkyl (wherein the optional substitution comprises from 1 to 3 substituents selected from halo, hydroxy, lower alkoxy, CN, NO₂, or optionally mono- or di-lower alkyl substituted amino)), and
   wherein
   R5 is aryl, aryl-lower alkyl, aryloxy, aroyl or N-heterocyclyl as defined above, and
   wherein R5 is optionally substituted by R7 which represents from 1 to 5 substitutents selected from halo, hydroxy, CN, NO₂ or oxo, or optionally substituted (lower-alkoxy, lower-alkyl, aryl, aryloxy, aroyl, lower-alkylsulphonyl, arylsulphonyl, optionally mono- or di-lower alkyl substituted amino, or N-heterocyclyl, or N-heterocyclyl-lower alkyl (wherein N-heterocyclyl is as defined above), and
   wherein R7 is optionally substituted by from 1 to 3 substitutents selected from halo, hydroxy, optionally mono- or di- lower-alkyl substituted amino, lower-alkyl carbonyl, lower-alkoxy or lower-alkylamido;
   R13 is lower alkyl, C3 to C10 cycloalkyl or C3-C10cycloalkyl-lower alkyl, all of which are independently optionally substituted by halo, hydroxy, CN, NO₂ or optionally mono- or di-lower alkyl-substituted amino; and
   R14 is H or optionally substituted (aryl, aryl-W-, aryl-lower alkyl-W-, C3 to C10 cycloalkyl, C3 to C10 cycloalkyl-W-, N-heterocyclyl or N-heterocyclyl-W- (wherein N-heterocyclyl is as defined above), phthalimide, hydantoin, oxazolidinone, or 2,6-dioxo-piperazine),
   wherein -W- is -O-, -C(O)-, -NH(R6)-, -NH(R6)-C(O)-, -NH(R6)-C(O)-O-, (where R6 is as defined above),-S(O)-, -S(O)₂- or -S-,
   wherein R14 is optionally substituted by R18 which represents from 1 to 10 substitutents selected from halo, hydroxy, CN, NO₂, oxo, amido, carbonyl, sulphonamido, lower-alkyldioxymethylene, or optionally substituted (lower-alkoxy, lower-alkyl, lower-alkenyl, lower alkynyl, lower alkoxy carbonyl, optionally mono- or di-lower alkyl substituted amino, aryl, aryl-lower alkyl, aryl-lower alkenyl, aryloxy, aroyl, lower-alkylsulphonyl, arylsulphonyl, N-heterocyclyl, N-heterocyclyl-lower alkyl (wherein N-heterocyclyl is as defined above), heterocyclyl or R14 comprising aryl has aryl fused with a hetero-atom containing ring, and
   wherein R18 is optionally substituted by R19 which represents from 1 to 4 substitutents selected from halo, hydroxy, CN, NO₂ or oxo, or optionally substituted (lower-alkoxy, lower-alkyl, lower-alkoxy-lower-alkyl, C₃-C₁₀cycloalkyl, lower-alkoxy carbonyl, halo-lower alkyl, optionally mono- or di-lower alkyl substituted amino, aryl, aryloxy, aroyl (e.g. benzoyl), acyl (e.g. lower-alkyl carbonyl), lower-alkylsulphonyl, arylsulphonyl or N-heterocyclyl, or N-heterocyclyl-lower alkyl (wherein N-heterocyclyl is as defined above)),
   wherein R19 is optionally substituted by from 1 to 4 substitutents selected from halo, hydroxy, CN, NO₂, oxo, optionally mono- or di-lower alkyl substituted amino, lower-alkyl, or lower-alkoxy;
b) a compound as disclosed in WO 00/69855, e.g. N2-(3-furanylcarbonyl)-L-norleucine-2(S)-methyl-4-oxotetrahydrofuran-3(R)-yl amide;
c) a compound as disclosed in WO 01/19796, WO 01/19808, WO 02/51983, WO 03/24923, WO 03/24924, WO 03/41649 or WO 03/42197, e.g. N-(2-(1-cyanocyclopropylamino)-1(R)-(2-benzylsulfonylmethyl)-2-oxoethyl)morpholine-4-carboxamide, N-(2-(cyanomethylamino)-1-(2-(difluoromethoxy)benzylsulfonylmethyl)-2-oxoethyl)pyridine-4-carboxamide, N-(2-(cyanomethylamino)-1(R)-(2-(difluoromethoxy)benzylsulfonylmethyl)-2-oxoethyl)-3,4-difluorobenzamide, N-(2-(cyanomethylamino)-1(R)-(2-(difluoromethoxy)benzylsulfonylmethyl)-2-oxoethyl)-3-methylbenzamide, N-(2-(cyanomethylamino)-1(R)-(2-(difluoromethoxy)benzylsulfonylmethyl)-2-oxoethyl)-1H-indole-5-carboxamide, N-(2-(cyanomethylamino)-1(R)-(2-(difluoromethoxy)benzylsulfonylmethyl)-2-oxoethyl)-5-methylthiophene-2-carboxamide, N-(2-(4-cyano-1-methylpiperidin-4-ylamino)-1(R)-(2-(difluoromethoxy)benzylsulfonylmethyl)-2-oxoethyl)morpholine-4-carboxamide, N-(2-(cyanomethylamino)-1(R)-(2-(difluoromethoxy)benzylsulfonylmethyl)-2-oxoethyl)-4-fluorobenzamide, N-(2-(cyanomethylamino)-1(R)-(2-(difluoromethoxy)benzylsulfinylmethyl)-2-oxoethyl)thiophene-3-carboxamide, N-(2-(cyanomethylamino)-1(R)-(2-(difluoromethoxy)benzylsulfonylmethyl)-2-oxoethyl)thiophene-2-carboxamide or N-(2-(cyanomethylamino)-1(R)-(2-(difluoromethoxy)benzylsulfonylmethyl)-2-oxoethyl)morpholine-4-carboxamide;
d) a compound as disclosed in WO 00/51998, WO 03/29200 or WO 03/37892, e.g. N-(1(S)-(N-(2-(benzyloxy)-1(R)-cyanoethyl)carbamoyl)-2-cyclohexylethyl)morpholine-4-carboxamide;
e) a compound as disclosed in WO 02/14314, WO 02/14315 or WO 02/14317, e.g. N1-(3-chloro-2-(4-(2-hydroxy-3-(5-(methylsulfonyl)-3-(4-(trifluoromethyl)phenyl)-4,5,6,7-tetrahydro-1H-pyrazolo(4,3-pyridin-1-yl)propyl)piperazin-1-yl)phenyl)-N3-methylurea, 1-(1-(3-(3-(4-bromophenyl)-5-(methylsulfonyl)-4,5,6,7-tetrahydro-1H-pyrazolo(4,3-c)pyridine-1-yl)-2-hydroxypropyl)piperidin-4-yl)-6-chloro-1,2,3,4-tetrahydroquinolin-2-one, or 1-(5-(methylsulfonyl)-3-(4-(trifluoromethyl)phenyl-4,5,6,7-tetrahydro-1H-pyrazolo(4,3-c)pyridine-1-yl)-3-(4-(6-(4-morpholinyl)-1H-pyrrolo(3,2-c)pyridine-3-yl)piperidin-1-yl)propan-2-ol;
f) a compound as disclosed in WO 01/89451, e.g. 5-(2-morpholin-4ylethoxy)benzofuran-2-carboxylic acid ((S)-3-methyl-1-((S)-3-oxo-1-(2-(3-pyridin-2-ylphenyl)-acetyl)azepan-4-ylcarbamoyl)butylamide;
g) a compound as disclosed in WO 02/32879, WO 01/09169 or WO 00/59881A1, e.g. N-(1-benzothien-2-ylcarbonyl)-N-(2-(2-fluorophenyl)-4-oxo-1,2,3,4-tetrahydropyrimidin-5-yl)-L-leucinamide;
h) a compound as disclosed in WO 00/48992, WO 00/49007 or WO 00/49008.

The term "mTOR inhibitor" as used herein includes, but is not limited to rapamycin (sirolimus) or a derivative thereof. Rapamycin is a known macrolide antibiotic produced by Streptomyces hygroscopicus. Suitable derivatives of rapamycin include e.g. compounds of formula A 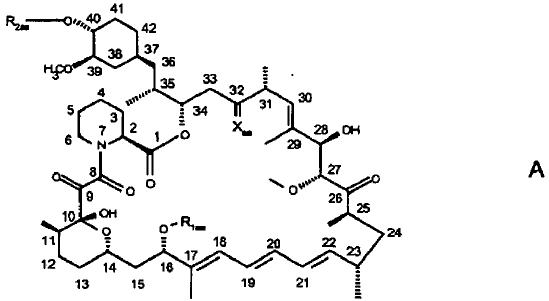 wherein
R₁ₐₐ is CH₃ or C₃₋₆alkynyl,
R₂ₐₐ is H or -CH₂-CH₂-OH, 3-hydroxy-2-(hydroxymethyl)-2-methyl-propanoyl or tetrazolyl, and
Xₐₐ is =O, (H,H) or (H,OH)
provided that R₂ₐₐ is other than H when Xₐₐ is =O and R₁ₐₐ is CH₃.
or a prodrug thereof when R₂ₐₐ is -CH₂-CH₂-OH, e.g. a physiologically hydrolysable ether thereof.

Compounds of formula A are disclosed e.g. in WO 94/09010, WO 95/16691, WO 96/41807, USP 5,362,718 or WO 99/15530 which are incorporated herein by reference. They may be prepared as diclosed or by analogy to the procedures described in these references.

Preferred rapamycin derivatives are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-0-(2-hydroxyethyl)-rapamycin. Further examples of rapamycin derivatives include e.g. CCI779 or 40- [3-hydroxy-2-(hydroxymethyl)-2-methylpropanoate]-rapamycin or a pharmaceutically acceptable salt thereof, as disclosed in USP 5,362,718, ABT578 or 40-(tetrazolyl)-rapamycin, particularly 40-epi-(tetrazolyl)-rapamycin, e.g. as disclosed in WO 99/15530, or rapalogs as disclosed e.g. in WO 98/02441 and WO01/14387, e.g. AP23573 or TAFA-93.

In one embodiment of the invention, 2-amino-2-[2-(4-C₂₋₂₀-alkyl-phenyl)ethyl]propane-1,3-diol compound, e.g. FTY720, FTY720 phosphate or, in each case, a pharmaceutically acceptable salt, e.g. a hydrochloride salt, thereof, is co-administered, e.g. concomitantly or in sequence, with at a least one interferon, as herein above described. For example, 2-amino-2-[2-(4-C₂₋₂₀-alkyl-phenyl)ethyl]propane-1,3-diol compound, e.g. FTY720, FTY720 phosphate or, in each case, a pharmaceutically acceptable salt is co-administered with a β-interferon, e.g. interferon beta-1a or interferon beta-1 b, e.g. administered by subcutaneous, intramuscular or oral routes.

The compounds used as active ingredients in the combinations of the invention can be prepared and administered as described in the cited documents, respectively. Also within the scope of this invention is the combination of more than two separate active ingredients as set forth above, i.e. a pharmaceutical combination within the scope of this invention could include three active ingredients or more. Further both the first agent and the co-agent are not the identical ingredient.

The administration of a pharmaceutical combination of the invention results not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit is that lower doses of the active ingredients of the combination of the invention can be used, for example, that the dosages need not only often be smaller but are also applied less frequently, which may diminish the incidence or severity of side-effects. This is in accordance with the desires and requirements of the patients to be treated.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

Utility of 2-amino-2-[2-(4-C₂₋₂₀-alkyl-phenyl)ethyl]propane-1,3-diol compound, e.g. FTY720 and FTY720-phosphate in treating, preventing or delaying the progression of multiple sclerosis in a paediatric patient or a patient suffering from a chronic condition, such as for example dyspnea, diarrhea, nausea, asthma may be demonstrated in clinic, for example in accordance with the methods hereinafter described.

Suitable clinical studies are, for example, open label, dose escalation studies in patients, e.g. children and adolescents of 10 to 18 years of age with multiple sclerosis. Such studies prove in particular the synergism of the active ingredients of the combination of the invention. The beneficial effects on multiple sclerosis can be determined directly through the results of these studies which are known as such to a person skilled in the art. Such studies are, in particular, suitable to compare the effects of a monotherapy using the active ingredients and a combination of the invention. Preferably, the dose of agent (a) is escalated until the Maximum Tolerated Dosage is reached, and the co-agent (b) is administered with a fixed dose. Alternatively, the agent (a) is administered in a fixed dose and the dose of co-agent (b) is escalated. Each patient receives doses of the agent (a) either daily or intermittent. The efficacy of the treatment can be determined in such studies, e.g., after 12, 18 or 24 weeks by evaluation of symptom scores every 6 weeks.

Alternatively, a placebo-controlled, double blind study can be used in order to prove the benefits of the invention mentioned herein.

The invention will now be described with reference to the following specific embodiments.

### Example 1

Micronized Compound A, e.g. 2-amino-2-[2-(4-octylphenyl)ethyl]propane-1,3-diol, hydrochloride salt (FTY720), is screened and 116.7 g of the screened compound is mixed with 9683.3 g mannitol (Parteck M200 from E. Merck). The mixture is then milled in a Frewitt MGI device (Key International Inc. USA) using a 30 mesh screen. Magnesium stearate Is screened using a 20 mesh screen and 200 g of the screened compound blended with the FTY720/mannitol mixture to produce a product composition.

The product composition is then compacted on a tablet press using a 7 mm die to form 120 mg tablets, each containing:

| | |
|---|---|
| Compound A, e.g. FTY720 * | 1.4 mg |
| Mannitol M200 | 116.2 mg |
| Magnesium stearate | 2.4 mg |
| | |
| Total | 120 mg |

| | |
|---|---|
| * 1 mg of Compound A in free form is equivalent to 1.12 mg of FTY720. | |

### Example 2

In a further example, the process of example 1 is repeated except that the magnesium stearate is replaced by Cutina® (hydrogenated castor oil).

### Example 3

Compound A, e.g. FTY720, and mannitol (Parteck M200 from E. Merck) are each screened separately using an 18 mesh screen. 1.9 g screened FTY720 is mixed with 40 g screened mannitol for 120 revolutions in a blender at 32 rpm. The FTY720/mannitol mixture is then screened through a 35 mesh screen.

The screened FTY720/mannitol mixture is added to a granulator along with a further 340.1 g mannitol and 12 g hydroxypropylcellulose. The mixture is mixed for 3 minutes. Water is then added at a rate of 100 ml/minute and the mixture granulated for 2 minutes. The granulation is transferred into a tray dryer and dried at 50 °C for 150 minutes.

The mixture is then milled in a Frewitt MGI device using a 35 mesh screen. Magnesium stearate is screened and 6 g of the screened compound is blended for 90 revolutions at 32 rpm with the FTY720/manntol mixture to produce a product composition showing a substantially uniform distribution of the compound throughout the mannitol in the blend.

The product composition is then filled into size 3 hard gelatin shells on an Hoflinger & Karg 400 encapsulation device. 120 mg of the product composition is added to each capsule. Therefore each capsule contains:

| | |
|---|---|
| FTY720 * | 0.56 mg |
| Mannitol M200 | 114.04 mg |
| Hydroxypropylcellulose | 3.6 mg |
| Magnesium stearate | 1.8 mg |
| | |
| Total | 120 mg |

### Example 4

Oral FTY720 1.25 or 5.0 mg, once-daily, reduced annualized relapse rate (ARR) by >50% and cumulative number of gadolinium-enhancing (Gd+) lesions by up to 80% versus placebo during a 6-month, placebo-controlled trial of 281 patients with relapsing multiple sclerosis (MS). All patients who subsequently opted to enter a long-term extension of the study received FTY720 once-daily for up to 36 months.

Patients entering the extension from the placebo group were re-randomized to fingolimod 1.25 mg or 5.0 mg; all other patients continued FTY720 treatment at their original dose (1.25 mg or 5.0 mg). During months 15 to 24, patients receiving FTY720 5.0 mg were switched to 1.25 mg.

Of the 250 patients who entered the extension, 173 completed month 36. The discontinuation rate during months 24-36 (8.0%) was notably lower than during months 12-24 (17.2%). After 36 months of continuous treatment, ARR remained low (0.20-0.21) and 68-73% of patients remained relapse-free. At month 36, the majority of patients in each group were free from Gd+ lesions (88-89%) or new T2 lesions (70-78%). The majority (76-80%) of patients were also free from 6-month sustained disability progression at month 36. The most frequently reported adverse events (AEs; >15% of patients) were nasopharyngitis, headache, fatigue, and influenza. Frequently reported AEs associated with FTY720 during months 0-6 (dyspnea, diarrhea, and nausea) were rarely reported during months 24-36 (1.1%, 2.7%, and 2.1%, respectively).

After 3 years of follow-up there was persistent inhibition of clinical and MRI activity in patients who received continuous oral FTY720 treatment at the 1.25 mg dose or the 5.0 mg dose. The 1.25 mg dose had a good safety profile.

Hamburg Quality of Life Questionnaire in MS (HAQUAMS) scores were recorded at baseline, month 3 (M3), and M6 during a core 6-month study, and M12 and M24 during an extension of the core study. The HAQUAMS consists of 38 items, 28 of which contribute to five sub-domains. Domain scores range from 1 to 5, with lower scores indicating improved HRQL. A total score was generated by averaging the five sub-domain scores.

Mean total scores at baseline were 1.9 for oral FTY720 1.25 mg, 5 mg and placebo; corresponding scores at M6 were 1.9 (mean change from baseline, -0.02), 1.9 (mean change, -0.01) and 2.0 (mean change, +0.12). Between group comparisons of change in total scores from baseline to M6 were favorable for 1.25mg-treated patients compared with placebo (p=0.044). HAQUAMS scores for specific domains were generally stable from baseline to M6 across all three treatment arms, though patients receiving oral FTY720 1.25 mg showed improvement in the fatigue/thinking domain compared with those on placebo (-0.05 vs +0.14; p=0.030). Stable total HAQUAMS scores were maintained for oral fingolimod-treated patients through M24.

### Example 5

An open-label, single-dose 28-day pharmacokinetic and safety/tolerability study of FTY720 in pediatric is performed (8 patients aged 11-17 years, 5 patients aged 6-10 years and 5 patients aged 1-5 years). The contents of 0.5 mg capsules is dispersed in water and administered as an oral solution at a dose of 0.07 mg/kg to a maximum dose of 5 mg. The dose is administered by study personnel either directly from the glass mixing vial or with an oral syringe followed by drinking 2 water-rinse cycles of the vial or syringe and an additional 50-200 mL of water.

Blood samples are collected for 28 days postdose and analyzed for fingolimod and fingolimod-phosphate blood concentrations by validated liquid chromatography methods with tandem mass spectrometry. Standard noncompartmental pharmacokinetic parameters are derived. Absolute lymphocyte counts are measured over 28 days to characterize the effect of FTY720 on lymphocyte decrease and recovery. Heart rate is recorded frequently for 24 hours postdose.

Safety and tolerability assessments includes information on adverse events, body height and weight, supine systolic and diastolic blood pressure and radial pulse rate; standard 12-lead electrocardiograms (ECGs) interpreted at eResearchTechnology, Inc (Philadelphia, PA, USA); standard hematology, biochemisty, and urinalysis parameters; screens for Hepatitis B surface antigen (HBsAg), Hepatitis C antibodies, and HIV; and pregnancy tests.

The results show that single-dose FTY720 is well tolerated in adolescents.

Lymphocyte responses: In adolescents, mean lymphocyte counts has decreased 85% from a predose count of 2.64 ± 1.04 x 10⁹/L to a nadir of 0.37 ± 0.17 x 10⁹/L at a median 2 days postdose. Lymphocyte counts is recovered thereafter back to baseline by the end-of-study visit on day 28. This pattern is similar to that previously measured in multiple studies in adults.

### Example 6

A 24-month, open-label (with blinded efficacy assessments), randomized, active-controlled, parallel-group, multicenter study is performed to evaluate the safety and efficacy of FTY720 on MRI measures of inflammation and clinical relapses in 118 children/adolescent patients (10 - 18 years old) with a relapsing-remitting course. The patients are randomized in a 1:1 ratio to receive either FTY720 administered orally once daily, or Avonex administered once weekly by intramuscular injection. The proportion of patients free of new/newly enlarging T2 MRI lesions at 2 years and relapse rate are assessed.

### Example 7

A randomized, double-blind, placebo-controlled, parallel, time-lagged, ascending, multiple oral dose study, is performed in patients with moderate asthma: 36 patients in 3 cohorts (12 patients per cohort). In each cohort, patients are randomized between the FTY720 group (9 patients) and placebo (3 patients)
Cohort 1: FTY720 0.5 mg or placebo
Cohort 2: FTY720 1.25 mg or placebo
Cohort 3: FTY720 2.5 mg or placebo
Duration of treatment: 10 days.
Patients continue to take their standard daily dose of long-acting ß2 agonist and inhaled corticosteroid throughout the study uninterrupted.
Pulmonary function test (FEV1, FEF25-75): is performed at 12-hour profile on Day 1 (predose, 1, 2, 3, 4, 5, 6, and 12hr post dose), 6-hour profile at baseline (Day -1) and on Day 10. An additional PFT is done 6 hours post dose on Days 2, 3, and 7.The results show that treatment initiation with FTY720 was well tolerated.

## Claims

1. A pharmaceutical composition comprising about 1.25 mg or less of a 2-amino-2-[2-(4-C₂₋₂₀ alkylphenyl)ethyl]propane-1,3-diol compound or a pharmaceutically acceptable salt thereof, for use in the treatment, prevention or delay of progression of multiple sclerosis in a patient, wherein the composition is to be administered orally and wherein the patient is a paediatric patient or is suffering from a chronic or recurrent condition e.g. selected from dyspnea, diarrhea, nausea and asthma.

2. A composition according to claim 1, which comprises about 1.25 mg of the compound.

3. A composition according to claim 1, which comprises about 0.5 mg or less of the compound.

4. A composition according to any preceding claim, wherein the patient is a paediatric patient.

5. Use of a 2-amino-2-[2-(4-C₂₋₂₀ alkylphenyl)ethyl]propane-1,3-diol compound or a pharmaceutically acceptable salt thereof, for the manufacture of a medicament for the treatment, prevention or delay of progression of multiple sclerosis in a patient, wherein the medicament is to be administered orally and wherein the patient is a paediatric patient or is suffering from a chronic or recurrent condition, e.g. selected from dyspnea, diarrhea, nausea and asthma.

6. A package comprising a pharmaceutical composition comprising about 1.25 mg, 0.5 mg or less of a 2-amino-2-[2-(4-C₂₋₂₀ alkylphenyl)ethyl]propane-1,3-diol compound or a pharmaceutically acceptable salt thereof; and a label bearing instructions to use of the composition for the treatment, prevention or delay of progression of multiple sclerosis in a patient, wherein the composition is to be administered orally and wherein the patient is a paediatric patient or is suffering from a chronic or recurrent condition, e.g. selected from dyspnea, diarrhea, nausea and asthma.

7. A method of treating, preventing or delaying the progression of multiple sclerosis in a patient, which comprises administering a pharmaceutical composition comprising about 1.25 mg, 0.5mg or less of a 2-amino-2-[2-(4-C₂₋₂₀ alkylphenyl)ethyl]propane-1,3-diol compound or a pharmaceutically acceptable salt thereof, wherein the composition is adminstered orally and wherein the patient is a paediatric patient or is suffering from a chronic or recurrent condition selected from dyspnea, diarrhea, nausea and asthma.

8. A composition, use, package or method according to any preceding claim, wherein the compound is administered once daily.

9. A composition, use, package or method according to any preceding claim, wherein the compound is administered with a co-agent which is selected from interferons, altered peptide ligands, immunosuppressants, adenosine deaminase inhibitors, IV immunoglobulin G, monoclonal antibodies to various T-cell surface markers, TH2 promoting cytokines, antispasticity agents, AMPA glutamate receptor antagonists, inhibitors of VCAM-1 expression or antagonists of its ligand, anti-Macrophage migration inhibitory factor (Anti-MIF), Cathepsin S inhibitors, mTor inhibitors.

10. A composition, use, package or method according to any preceding claim, wherein the compound is 2-amino-2-[2-(4-octylphenyl)ethyl] propane-1,3-diol, in free form or in a pharmaceutically acceptable salt, or FTY720 phosphate, for example is 2-amino-2-[2-(4-octylphenyl)ethyl] propane-1,3-diol hydrochloride.
